# EUROPEAN PATENT SPECIFICATION

(11) **EP 1 894 927 B1**
(45) Date of publication and mention of the grant of the patent: **10.11.2010**
(21) Application number: 06018236.7
(22) Date of filing: 31.08.2006
(51) Int. Cl.: C07D 403/12

(54) **The use of moxonidine salts for purification of moxonidine**
Verwendung der Monoxidinssalze zur Reinigung von Monoxidin
Utilisation des sels de moxonidine pour la purification de monoxidine

(43) Date of publication of application: 05.03.2008
(73) Proprietor: CHEMAGIS LTD., 51200 Bnei-Brak (IL)
(72) Inventor: Naddaka, Vladimir, 49400 Petach-Tikva (IL); Klopfer, Eyal, 64955 Tel Aviv (IL); Saeed, Shady, 33266 Haifa (IL); Adin, Itai, 84684 Beer Sheva (IL); Iustain, Carmen, 84750 Beer Sheva (IL); Arad, Oded, 76303 Rechovor (IL); Kaspi, Joseph, 53201 Givatayim (IL)
(74) Representative: Albrecht, Thomas

(56) References cited:
- WO-A-00/44355
- WO-A-97/46241
- US-A- 4 323 570
- CZESKIS B A: "Synthesis of triple [14C]-labeled moxonidine" JOURNAL OF LABELLED COMPOUNDS AND RADIOPHARMACEUTICALS, JOHN WILEY, CHICHESTER, GB, vol. 47, no. 10, 2004, pages 699-704, XP002394964 ISSN: 0362-4803

## Description

### FIELD OF THE INVENTION

The present invention relates to a novel purification process of the drug Moxonidine.

### BACKGROUND OF THE INVENTION

Moxonidine (4-chloro-5-(imidazoline-2-ylamino)-6-methoxy-2-methylpyrimidine), has the structural formula (I) below and is used as an antihypertensive drug.

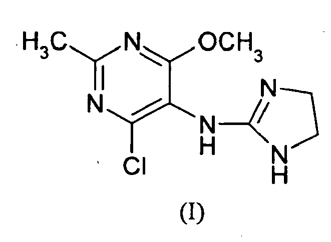

Moxonidine was approved for use in Germany in 1991 and is currently commercially available in Europe, e.g., in Germany, Austria and in the UK.

U.S. patent No. 4,323,570 (hereinafter the '570 patent) describes a method of preparing Moxonidine (I) by reacting 4,6-dichloro-2-methyl-5-(1-acetyl-2-imidazolin-2-yl)-aminopyrimidine (hereinafter DMAIA) with about 2 equivalents of sodium methoxide in methanol under reflux. It is stated in example 3 of the '570 patent that Moxonidine is obtained by crystallization from nitromethane having a melting point of 217-219°C. The hydrochloride salt of Moxonidine is described in example 25, having a melting point of 189°C.

Other inorganic as well as organic Moxonidine salts are described e.g., in application WO 97/46241 as being useful in treating congestive heart failure, in application WO 00/44355 wherein low solubility Moxonidine and salts thereof may be used for treating hypertension, and in patent US 4952410 wherein pharmaceutical composition containing Moxonidine or its pharmaceutically acceptable salts and hydrochlorothiazide is recited.

Nitromethane, the solvent from which Moxonidine is recrystallized, is a hazardous and explosive solvent in addition to being toxic (the allowed limit of nitromethane in human drugs, according to ICH guideline is merely 50 ppm). It is incompatible with many commonly used industrial reagents e.g., amines, strong acids and strong bases. Therefore, the crystallization from nitromethane is not suitable for industrial implementation due to its hazardous nature. Its toxicity renders it not suitable for use in human drugs. Thus there is a need in the art for an alternative purification process of Moxonidine using safer and more environmentally friendly solvents.

According to the teaching of the European patent application titled "Novel purification process of Moxonidine", substantial levels of the impurities 4,6-dimethoxy-2-methyl-5-(2-imidazolin-2-yl)-aminopyrimidine of formula (II) and 4,6-dichloro-2-methyl-5-(2-imidazolin-2-yl)-aminopyrimidine of formula (III) are obtained during the synthesis of Moxonidine as described in the reference examples 1 and 2 respectively.

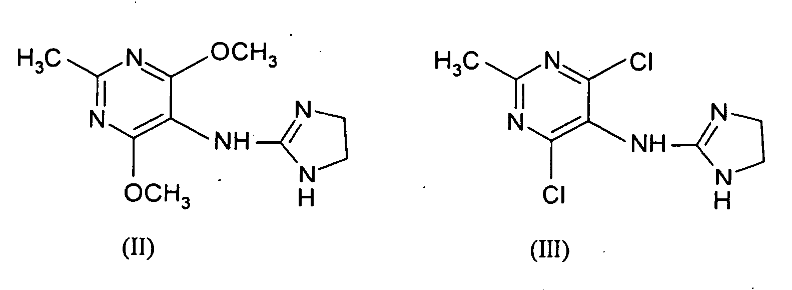

### BRIEF SUMMARY OF THE INVENTION

The inventors of the present invention have prepared salt forms of 4-chloro-5-(imidazoline-2-ylamino)-6-methoxy-2-methyl-pyrimidine, also known by the name Moxonidine. The said salt forms are obtained in high purity and yield and are prepared by methods known in the art for preparing acid addition salts of active pharmaceutical ingredients e.g., by treating the active pharmaceutical ingredient with an acid to obtain its salt form.

While searching for processes for purifying Moxonidine, the inventors of the present invention have surprisingly discovered a simple and effective process for purifying crude Moxonidine via the said crystalline salt forms of Moxonidine, as described in detail herein.

According to another embodiment of the present invention, the process for purifying Moxonidine via a crystalline Moxonidine salt comprises:
reacting crude Moxonidine with an acid in an organic solvent;
isolating the Moxonidine salt from the reaction mixture and optionally
purifying the salt by crystallization;
reacting the Moxonidine salt with a base; and
optionally crystallizing Moxonidine base from an organic solvent.

According to another embodiment of the present invention, suitable acids are e.g., inorganic acids or organic acids, wherein suitable organic acids are selected from the group consisting of oxalic acid, formic acid, maleic acid, fumaric acid, malonic acid, trifluoroacetic acid, benzoic acid, chloroacetic acid, and combinations thereof.

According to another embodiment of the present invention, the process provided herein enables the preparation of purified Moxonidine in at least 85% yield.

According to another embodiment of the present invention, the process provided herein enables obtaining the purified Moxonidine having a purity of at least 98%, preferably having a purity equal to or greater than 99.5%, and more preferably having a purity equal to or greater than 99.8%, according to HPLC.

According to another embodiment of the present invention, the process provided herein enables obtaining the purified Moxonidine containing less than about 0.06% of 4,6-dimethoxy-2-methyl-5-(2-imidazolin-2-yl)-aminopyrimidine (impurity II), and/or less than about 0.14% of 4,6-dichloro-2-methyl-5-(2-imidazolin-2-yl)-aminopyrimidine (impurity III), according to HPLC.

### BRIEF DESCRIPTION OF THE FIGURES

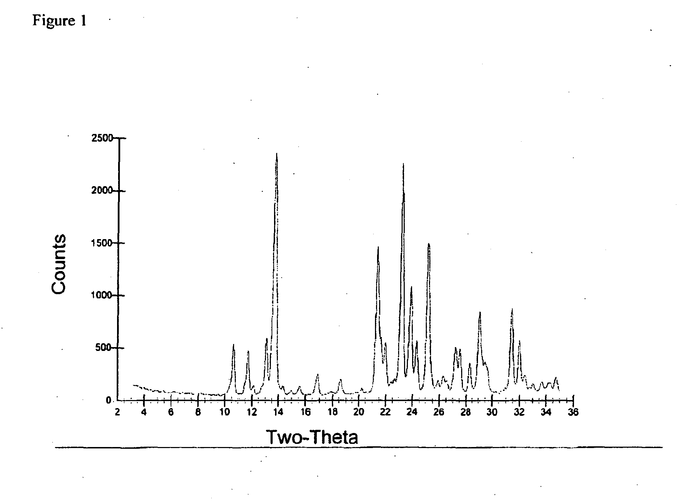

Figure 1 depicts the powder X-ray diffraction pattern of the crystalline mono-Moxonidine oxalate salt.

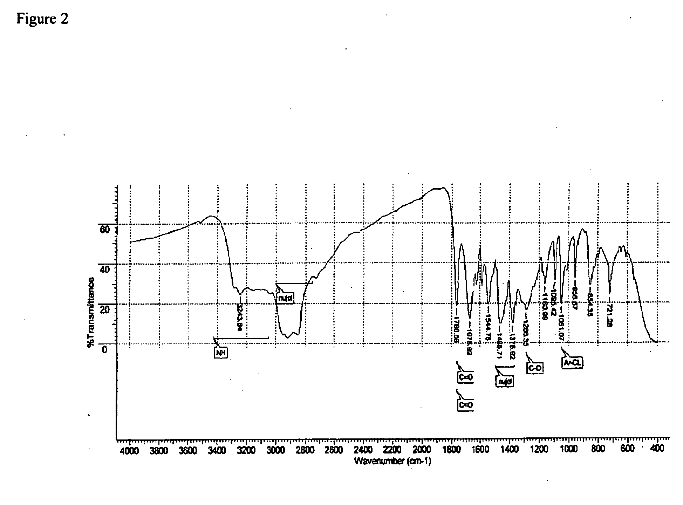

Figure 2 depicts the infra-red (IR) spectrum of the crystalline mono-Moxonidine oxalate salt.

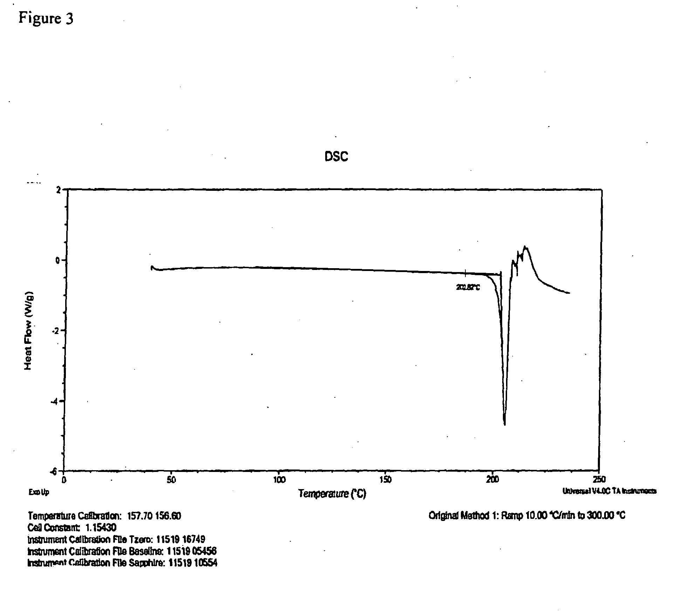

Figure 3 depicts the differential scanning calorimetry (DSC) curve of the crystalline mono-Moxonidine oxalate salt.

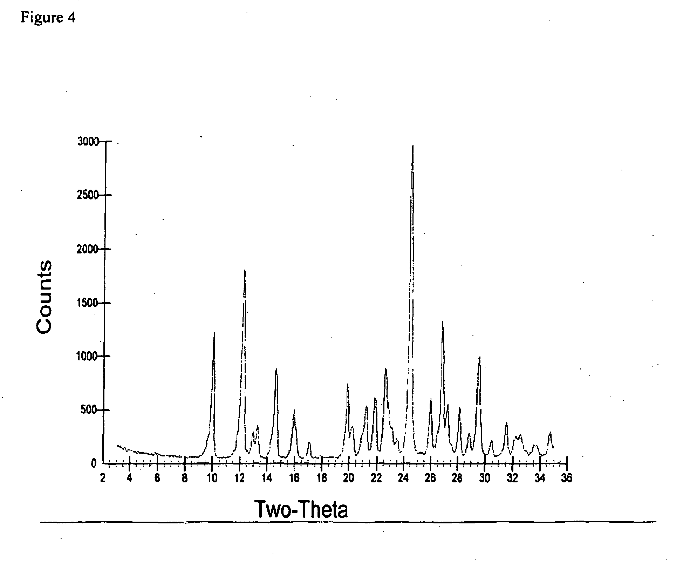

Figure 4 depicts the powder X-ray diffraction pattern of the crystalline mono-Moxonidine formate salt.

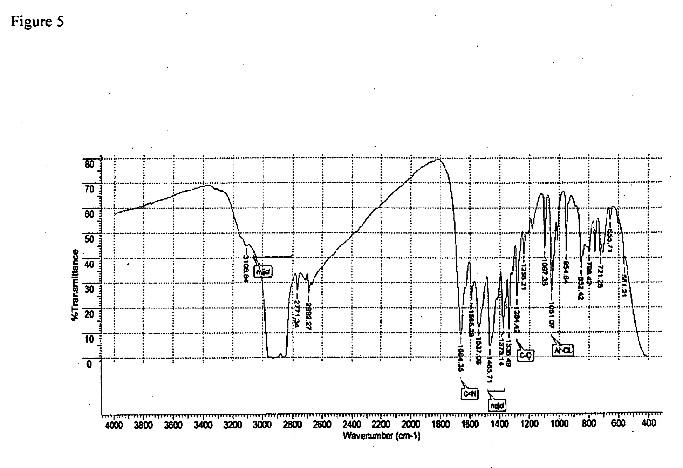

Figure 5 depicts the infra-red (IR) spectrum of the crystalline mono-Moxonidine formate salt.

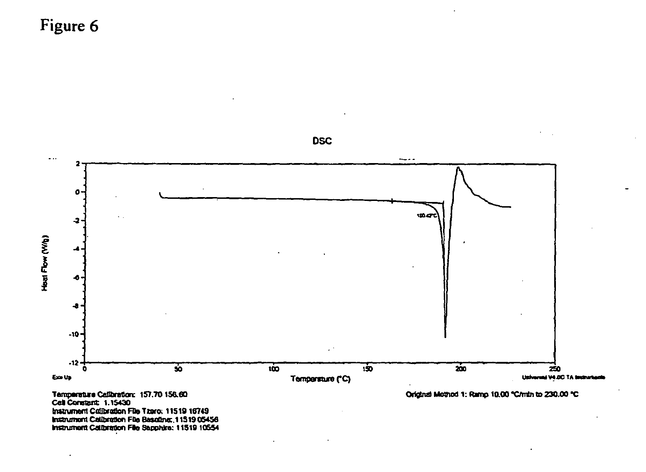

Figure 6 depicts the differential scanning calorimetry (DSC) curve of the crystalline mono-Moxonidine formate salt.

### DESCRIPTION OF THE PREFERRED EMBODIMENTS

The inventors of the present invention have prepared salt forms of 4-chloro-5-(imidazoline-2-ylamino)-6-methoxy-2-methyl-pyrimidine, also known by the name Moxonidine. The said salt forms are obtained in high purity and yield and are prepared by methods known in the art for preparing acid addition salts of active pharmaceutical ingredients e.g., by treating the active pharmaceutical ingredient with an acid to obtain its salt form.

While searching for processes for purifying Moxonidine, the inventors of the present invention have surprisingly discovered a simple and effective process for purifying crude Moxonidine *via* the said crystalline salt forms of Moxonidine, as described in detail herein. It may be understood by the skilled artisan that separating the impurity of formula II or the impurity of formula III (which are referred to herein also as impurity II or impurity III respectively), and specially impurity II, from Moxonidine is not an easy task to achieve due to the close similarities between structures of these molecules.

According to another embodiment of the present invention, the process for purifying Moxonidine via a crystalline Moxonidine salt comprises:
reacting crude Moxonidine with an acid in an organic solvent;
isolating the Moxonidine salt from the reaction mixture and optionally
purifying the salt by crystallization;
reacting the Moxonidine salt with a base; and
optionally crystallizing Moxonidine base from an organic solvent.

According to another embodiment of the present invention, suitable acids are e.g., inorganic acids or organic acids, wherein suitable organic acids are selected from the group consisting of oxalic acid, formic acid, maleic acid, fumaric acid, malonic acid, trifluoroacetic acid, benzoic acid, chloroacetic acid, and combinations thereof.

According to another embodiment of the present invention, suitable solvents for precipitating the Moxonidine salt prior to its isolation are selected from the group consisting of methanol, ethanol, 1-propanol, 2-propanol, 1-butanol, dimethyl sulfoxide (DMSO), N,N-dimethylformamide (DMF), N,N-dimethylacetamide (DMA), N-methyl-2-pyrrolidinone (NMP), ethyl acetate, diethyl ether, tert-butyl methyl ether, toluene, water and mixtures thereof.

According to another embodiment of the present invention, the base is an organic base or an inorganic base, wherein inorganic bases are selected from the group consisting of sodium hydroxide, potassium hydroxide, sodium bicarbonate, potassium bicarbonate, sodium carbonate, potassium carbonate, and combinations thereof.

According to another embodiment of the present invention, the crystallization solvent of the present process is selected from the group consisting of methanol, ethanol, 1-propanol, 2-propanol, 1-butanol, 2-butanol, 2-methyl-1-butanol, 3-methyl-1-propanol, n-amyl-alcohol, dimethyl sulfoxide (DMSO), N,N-dimethylformamide (DMF), N,N-dimethylacetamide (DMA), N-methyl-2-pyrrolidone (NMP), and mixtures thereof.

In another embodiment, Moxonodine can be crystallized, according to the process of the present invention, from a mixture of a high boiling point solvent e.g., dimethyl sulfoxide (DMSO) and an acid, wherein a suitable acid can be either an inorganic acid or an organic acid, e.g., formic acid, acetic acid or propionic acid.

According to another embodiment of the present invention, the solvent used for washing the obtained crystals is selected from the group consisting of methanol, ethanol, 1-propanol, 2-propanol, acetone, water, and mixtures thereof.

According to another embodiment of the present invention, the process provided herein enables the preparation of purified Moxonidine in at least 85% yield.

According to another embodiment of the present invention, the process provided herein enables obtaining the purified Moxonidine having a purity of at least 98%, preferably having a purity equal to or greater than 99.5%, and more preferably having a purity equal to or greater than 99.8%, according to HPLC.

According to another embodiment of the present invention, the process provided herein enables obtaining the purified Moxonidine containing less than about 0.06% of 4,6-dimethoxy-2-methyl-5-(2-imidazolin-2-yl)-aminopyrimidine ( impurity II) and/or less than about 0.14% of 4,6-dichloro-2-methyl-5-(2-imidazolin-2-yl)-aminopyrimidine (impurity III), according to HPLC.

Reference is now made to the following examples, which together with the above descriptions illustrate the invention in a non-limiting fashion. Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

HPLC measurements of Moxonidine samples were performed using HPLC system, equipped with Phenomenex Luna 5µ C8(2) (4.6 x 250 mm) column, and a UV detector operated on 230 nm. Analyses were performed using the following mobile phase, at flow rate of 1.2 ml/minute. Eluent A: 10 mM pentanesulfonic acid, pH = 3.0 with H₂SO₄. Eluent B: acetonitrile. Gradient (A/B, v/v): 0 min (94/6), 4 min (94/6), 20 min (64/36), 50 min (64/36).

Moxonidine crystalline salt forms were characterized by powder X-ray diffraction, thereby generating fingerprint powder X-ray diffraction patterns for each particular crystalline form. Measurements of 2θ values typically are accurate to within ± 0.2 degrees 2θ. X-ray diffraction data were acquired using a PHILIPS X-ray diffractometer model PW1050-70. System description: Kα1=1.54178 Å, voltage 40kV, current 28 mA, diversion slit = 1°, receiving slit = 0.2 mm, scattering slit = 1° with a Graphite monochromator. Experiment parameters: pattern measured between 2θ = 4° and 2θ = 30° with 0.05° increments; count time was 0.5 second per increment.

Moxonidine crystalline salt forms of the present invention were further characterized by Fourier-transform infra-red (FTIR) spectroscopy to an accuracy of ± 4 cm⁻¹ using a Nicolet Fourier-Transform infra-red spectrometer model Avatar 360, with Omnic software version 5.2. All samples were run using KBr pellets. FTIR is a well-known spectroscopic analytical tool, which measures the absorption of IR energy by a sample from transitions in molecular vibrational energy levels. While FTIR is primarily used for identification of functional groups in a molecule, different polymorphic forms also can exhibit differences in FTIR.

Moxonidine crystalline salt forms of the present invention also were characterized by differential scanning calorimetry (DSC), run on TA instruments model Q1000, with Universal software version 3.88. Samples were analyzed inside crimped 40 µl aluminum pans. Heating rate for all samples was 5 °C/min. Differential scanning calorimetry (DSC) graphs were recorded using a TA Instruments Q1000 Thermal Analyzer with Universal software (version 3.88). Samples were analyzed inside crimped 40 µl aluminum pans at a heating rate of 5 °C/min.

### Reference Example 1 - Preparation of Moxonidine by reaction of 4,6-dichloro-2-methyl-5-(1-acetyl-2-imidazolin-2-yl)-aminopyrimidine (DMAIA) with sodium methoxide according to the teaching of the '570 patent.

4,6-dichloro-2-methyl-5-( -acetyl -2 -imidazolin-2 -yl)-aminopyrimi dine, (DMAIA), (10 g, 0.0347 mol) was mixed with a solution of sodium methoxide (3.78 g, 0.07 mol, 2.02 equiv.) in 35 ml of methanol and boiled for 2 hours. Then, water (100 ml) was added and the reaction mixture was cooled to ambient temperature. A colorless product was collected by filtration, washed with water and dried at 50° C overnight to yield crude Moxonidine [containing 6.5% of impurity II and 0.61% of impurity III].

### Reference Example 2 - Preparation of Moxonidine by reaction of DMAIA with potassium carbonate according to the teaching of the '649 patent.

Potassium carbonate (9.6 g, 0.0694 mol, 2 molar equiv.) was added to a suspension of DMAIA (10 g, 0.0347 mol) in methanol (80 ml) and the mixture was heated at 47-52° C for 5 hours. Then, the reaction mixture was cooled to ambient temperature and acetic acid (10 ml) and water (70 ml) were added. After stirring for half an hour, 25% solution of ammonium hydroxide (10 ml) was added and the mixture was stirred for one hour. A colorless precipitate was collected by filtration, washed with water and dried at 50° C overnight to yield crude Moxonidine [containing 0.07% of impurity II and 0.40% of impurity III].

### Example 1 - Preparation of Moxonidine base via mono-Moxonidine oxalate

A mixture of crude Moxonidine [3 g, containing 0.61 % of impurity II and 1.5% of impurity III], oxalic acid dihydrate (1.8 g, 1.1 equiv.), 1-propanol (18 ml) and methanol (9 ml) was heated under reflux to obtain a clear solution. The solution was cooled in an ice-bath for 3.5 hours. Then, the thus formed colorless crystals were collected by filtration, washed with 1-propanol and dried at 50° C overnight to obtain 3.6 g of mono-Moxonidine oxalate in 87.0% yield [containing 0.04% of impurity II and 1.5% of impurity III].

20% aqueous NaOH solution was added at ambient temperature to a suspension of mono-Moxonidine oxalate (3.6 g) in water (18 ml) to produce a pH of 10-11. The mixture was stirred for one hour and the precipitate was collected by filtration, washed with water and 2-propanol and dried at 50° C overnight to yield 2.48 g of Moxonidine base in 94.0% yield, [containing 0.04% of impurity II and 1.25% of impurity III].

A mixture of 2.48 g of Moxonidine base and 12.5 ml of DMSO was heated at 90° C to obtain a clear solution. The solution was cooled to room temperature and kept at this temperature for about 3 hours. A colorless precipitate was collected by filtration, washed with water and 2-propanol and dried at 50° C overnight to afford 2.11 g of pure Moxonidine base in 85.0% yield having purity of 99.8%, [containing 0.05% of impurity II and 0.13% of impurity III].

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A process for purifying Moxonidine via a crystalline Moxonidine salt, the process comprising:
reacting crude Moxonidine with an acid in an organic solvent;
isolating the Moxonidine salt from the reaction mixture and optionally purifying the salt by crystallization;
reacting the Moxonidine salt with a base; and
optionally crystallizing Moxonidine base from an organic solvent.

2. The process of claim 1, wherein suitable acids are inorganic acids or organic acids, wherein suitable organic acids are selected from the group consisting of oxalic acid, formic acid, maleic acid, fumaric acid, malonic acid, trifluoroacetic acid, benzoic acid, chloroacetic acid, and combinations thereof.

3. The process of claim 1, wherein a suitable solvent for precipitating the Moxonidine salt prior to its isolation is selected from the group consisting of methanol, ethanol, 1-propanol, 2-propanol, 1-butanol, dimethyl sulfoxide (DMSO), N,N-dimethylformamide (DMF), N,N-dimethylacetamide (DMA), N-methyl-2-pyrrolidinone (NMP), ethyl acetate, diethyl ether, tert-butyl methyl ether, toluene, water, and mixtures thereof.

4. The process of claim 1, wherein the base is an organic base or an inorganic base, wherein inorganic bases are selected from the group consisting of sodium hydroxide, potassium hydroxide, sodium bicarbonate, potassium bicarbonate, sodium carbonate, potassium carbonate, and combinations thereof.

5. The process of claim 1, wherein the solvent used for washing the obtained crystals is selected from the group consisting of methanol, ethanol, 1-propanol, 2-propanol, acetone, water, and mixtures thereof.

6. The process of claim 1, wherein the purified Moxonidine is obtained in a yield of at least 85%.

7. The process of claim 1, wherein the purified Moxonidine is obtained having a purity of at least 98%.

8. The process of claim 7, wherein the purified Moxonidine is obtained having a purity of 99.8%.

9. The process of claim 1, wherein the purified Moxonidine contains less than 0.06% of 4,6-dimethoxy-2-methyl-5-(2-imidazolin-2-yl)-aminopyrimidine (impurity II).

10. The process of claim 1, wherein the purified Moxonidine contains less than 0.14% of 4,6-dichloro-2-methyl-5-(2-imidazolin-2-yl)-aminopyrimidine (impurity III).

11. The process of claim 1, wherein the purified Moxonidine contains less than 0.06% of 4,6-dimethoxy-2-methyl-5-(2-imidazolin-2-yl)-aminopyrimidine (impurity II) and less than 0.14% of 4,6-dichloro-2-methyl-5-(2-imidazolin-2-yl)-aminopyrimidine (impurity III).

## Patentansprüche

1. Verfahren zur Reinigung von Moxonidin über ein kristallines Moxonidinsalz, wobei das Verfahren umfasst:
Umsetzen von rohem Moxonidin mit einer Säure in einem organischen Lösungsmittel;
Isolieren des Moxonidinsalzes aus der Reaktionsmischung und gegebenenfalls Reinigen des Salzes durch Kristallisation;
Umsetzen des Moxonidinsalzes mit einer Base; und
gegebenenfalls Kristallisieren der Moxonidinbase aus einem organischen Lösungsmittel.

2. Verfahren gemäß Anspruch 1, wobei geeignete Säuren anorganische oder organische Säuren sind, wobei geeignete organische Säuren ausgewählt sind aus der Gruppe, bestehend aus Oxalsäure, Ameisensäure, Maleinsäure, Fumarsäure, Malonsäure, Trifluoressigsäure, Benzoesäure, Chloressigsäure und Kombinationen davon.

3. Verfahren gemäß Anspruch 1, wobei ein für das Ausfällen des Moxonidinsalzes vor seiner Isolierung geeignetes Lösungsmittel ausgewählt ist aus der Gruppe, bestehend aus Methanol, Ethanol, 1-Propanol, 2-Propanol, 1-Butanol, Dimethylsulfoxid (DMSO), N,N-Dimethylformamid (DMF), N,N-Dimethylacetamid (DMA), N-Methyl-2-pyrrolidinon (NMP), Ethylacetat, Diethylether, tert-Butylmethylether, Toluol, Wasser und Mischungen davon.

4. Verfahren gemäß Anspruch 1, wobei die Base eine organische Base oder eine anorganische Base ist, wobei anorganische Basen ausgewählt sind aus der Gruppe, bestehend aus Natriumhydroxid, Kaliumhydroxid, Natriumbicarbonat, Kaliumbicarbonat, Natriumcarbonat, Kaliumcarbonat und Kombinationen davon.

5. Verfahren gemäß Anspruch 1, wobei das zum Waschen der erhaltenen Kristalle verwendete Lösungsmittel ausgewählt ist aus der Gruppe, bestehend aus Methanol, Ethanol, 1-Propanol, 2-Propanol, Aceton, Wasser und Mischungen davon.

6. Verfahren gemäß Anspruch 1, wobei das gereinigte Moxonidin in einer Ausbeute von mindestens 85% erhalten wird.

7. Verfahren gemäß Anspruch 1, wobei das gereinigte Moxonidin mit einer Reinheit von mindestens 98% erhalten wird.

8. Verfahren gemäß Anspruch 7, wobei das gereinigte Moxonidin mit einer Reinheit von 99,8% erhalten wird.

9. Verfahren gemäß Anspruch 1, wobei das gereinigte Moxonidin weniger als 0,06% 4,6-Dimethoxy-2-methyl-5-(2-imidazolin-2-yl)-aminopyrimidin (Verunreinigung II) enthält.

10. Verfahren gemäß Anspruch 1, wobei das gereinigte Moxonidin weniger als 0,14% 4,6-Dichlor-2-methyl-5-(2-imidazolin-2-yl)-aminopyrimidin (Verunreinigung III) enthält.

11. Verfahren gemäß Anspruch 1, wobei das gereinigte Moxonidin weniger als 0,06% 4,6-Dimethoxy-2-methyl-5-(2-imidazolin-2-yl)-aminopyrimidin (Verunreinigung II) und weniger als 0,14% 4,6-Dichlor-2-methyl-5-(2-imidazolin-2-yl)-aminopyrimidin (Verunreinigung III) enthält.

## Revendications

1. Procédé de purification de la moxonidine via un sel cristallin de moxonidine, ledit procédé comprenant les étapes consistant à
- faire réagir de la moxonidine crue avec un acide dans un solvant organique;
- isoler le sel de moxonidine à partir du mélange de réaction, et éventuellement
- purifier ledit sel de moxonidine avec une base, et éventuellement
- cristalliser la base de moxonidine à partir d'un solvant organique.

2. Procédé selon la revendication 1, dans lequel des acides appropriés sont des acides organiques ou des acides inorganiques, les acides organiques étant choisis parmi le groupe consistant en acide oxalique, acide formique, acide maléique, acide fumarique, acide malonique, acide trifluoroacétique, acide benzoïque, acide chloracétique et combinaisons de ceux-ci.

3. Procédé selon la revendication 1, dans lequel un solvant approprié est choisi parmi le groupe consistant en méthanol, éthanol, propanol-1, propanol-2, butanol-1, diméthylsulfoxyde (DMSO), diméthylformamide (DMF), N,N-diméthylacétamide (DMA), N-méthyl-pyrrolidone (NMP), acétate d'éthyle, diéthyléther, méthyltertio-butyléther, toluène, eau et mélanges de ceux-ci.

4. Procédé selon la revendication 1, dans lequel la base est une base organique ou une base inorganique, des bases inorganiques étant choisies parmi le groupe consistant en hydroxyde de sodium, hydroxyde de potassium, bicarbonate de sodium, bicarbonate de potassium, carbonate de sodium, carbonate de potassium et combinaisons de ceux-ci.

5. Procédé selon la revendication 1, dans lequel le solvant qui est utilisé pour laver les cristaux obtenus, est choisi parmi le groupe consistant en méthanol, éthanol, propanol-1, propanol-2, acétone, eau et mélanges de ceux-ci.

6. Procédé selon la revendication 1, dans lequel la moxonidine purifiée est obtenue avec un rendement d'au moins 85%.

7. Procédé selon la revendication 1, dans lequel la moxonidine purifiée obtenue présente une pureté de 98%.

8. Procédé selon la revendication 7, dans lequel la moxonidine purifiée obtenue présente une pureté de 99,8%.

9. Procédé selon la revendication 1, dans lequel la moxonidine purifiée contient moins de 0,06% de 4,6-diméthoxy-2-méthyl-5-(2-imidazolin-2-yl)-aminopyrimidine (l'impureté II).

10. Procédé selon la revendication 1, dans lequel la moxonidine purifiée contient moins de 0,14% de 4,6-dichloro-2-méthyl-5-(2-imidazolin-2-yl)-aminopyrimidine (l'impureté III).

11. Procédé selon la revendication 1, dans lequel la moxonidine purifiée contient moins de 0,06% de 4,6-diméthoxy-2-méthyl-5-(2-imidazolin-2-yl)-aminopyrimidine (l'impureté II) et moins de 0,14% de 4,6-dichloro-2-méthyl-5-(2-imidazolin-2-yl)-aminopyrimidine (l'impureté III).
